# FASCICULE DE BREVET EUROPEEN

(11) **EP 1 470 805 B1**
(45) Date de publication et mention de la délivrance du brevet: **23.01.2008**
(21) Numéro de dépôt: 04290750.1
(22) Date de dépôt: 19.03.2004
(51) Int. Cl.: A61F 2/46, A61B 17/15

(54) **Mesureur de taille à utilisation simplifiée**
Vereinfachtes Grössenmessgerät
Simplified size measuring device

(30) Priorité: 24.04.2003 FR 0305061
(43) Date de publication de la demande: 27.10.2004
(73) Titulaire: Aesculap, 52000 Chaumont (FR)
(72) Inventeur: Plumet, Sylvie, 52000 Chaumont (FR); Moussa, Said, 52000 Chamarandes-Choignes (FR)
(74) Mandataire: Eidelsberg, Olivier Nathan

(56) Documents cités:
- US-A- 5 417 694
- US-A- 6 013 081
- PATENT ABSTRACTS OF JAPAN vol. 1997, no. 03, 31 mars 1997 (1997-03-31) -& JP 08 308857 A (NAKASHIMA PROPELLER KK), 26 novembre 1996 (1996-11-26)

## Description

La présente invention se rapporte à un mesureur de taille, c'est-à-dire un dispositif permettant de déterminer, parmi plusieurs (en un nombre fini cependant) tailles d'implants disponibles, la taille d'implant fémoral optimale à implanter dans un fémur. Le document US-A-6013081 représente l'état de la technique le plus proche et détermine le préambule de la revendication 1.

Aujourd'hui, pour déterminer la bonne taille, le chirurgien doit avoir à disposition l'ensemble des implants dans les différentes tailles et évaluer la taille optimale du fémur avant la résection. En outre, il lui est difficile de connaître l'incidence du choix de la taille de l'implant sur la coupe de l'os et le position de l'implant.

La présente invention vise à surmonter ces inconvénients en proposant un mesureur de taille qui permet au chirurgien de déterminer très simplement et très rapidement, parmi un nombre donné fini de tailles d'implant fémoral, dans un premier temps quelle taille correspond exactement au fémur sur lequel doit être implanté l'implant fémoral, et, dans un deuxième temps, s'il n'y a pas, parmi les tailles disponibles, de taille correspondant exactement à celle du fémur, laquelle s'en approche le plus, et l'endroit exact où doit être réalisée la résection antérieure suivant l'axe fémoral pour obtenir un positionnement parfait de l'implant sur la corticale antérieure, gage d'un bon positionnement de la trochlée de l'implant et réduisant les surtensions patellaires in vivo, tout en informant l'épaisseur coupée en plus ou en moins au niveau des condyles postérieurs.

Suivant l'invention, le mesureur de taille, comportant un corps principal ayant un palpeur agencé de manière mobile par rapport au corps principal de sorte qu'un pointeur principal soit déplacé en fonction du réglage en face d'une plage comportant plusieurs repères correspondants aux tailles d'implant disponibles, est caractérisé en ce qu'il comporte un coulisseau monté mobile par rapport au corps principal, le coulisseau comportant un premier pointeur et un deuxième pointeur, le premier pointeur étant agencé de manière à être déplacé, lors du coulissement du coulisseau, en face de ladite plage, et le deuxième coulisseau se déplaçant, lors du mouvement du coulisseau, en face de gradations disposées sur une région fixe par rapport au corps principal, la distance entre les deux pointeurs du coulisseau étant telle que lorsque le premier pointeur est en face d'un des repères correspondant à l'une des tailles d'implant disponibles, le deuxième pointeur indique sur les gradations la hauteur, par exemple en millimètres, de la coupe à réaliser en postérieur afin que l'implant affleure parfaitement au niveau de la corticale antérieure.

Ainsi, le chirurgien, pour déterminer le bon implant parmi plusieurs tailles d'implant, place les condyles sur le plateau, ajuste le palpeur en affleurement sur la corticale antérieure, lit en face du pointeur principal la taille à choisir, et si le pointeur ne se trouve pas exactement en face d'une des tailles disponibles, il fait alors coulisser le coulisseau pour amener le premier pointeur en face de l'une des tailles, de préférence la taille plus petite se trouvant la plus proche du pointeur principal, le deuxième pointeur indiquant alors, sur les gradations formées sur le corps principal, la hauteur de résection supplémentaire de l'os au niveau des condyles postérieurs, pour une adaptation parfaite de la taille choisie. La procédure est ainsi très simplifiée, sans avoir à changer de position le mesureur et simplement en faisant coulisser, par exemple par une clé à molette, le coulisseau.

De préférence, la plage se trouve sur une plaque, solidaire du palpeur et sensiblement de forme plane pour s'adapter à un fémur dont la coupe antéro-postérieure a déjà été réalisée.

On obtient ainsi un système particulièrement simple d'emploi.

De préférence, le corps principal comporte un plateau de réception des condyles, disposé en face du palpeur.

La mesure est alors très simple à effectuer.

La présente invention vise également un procédé de mesure du fémur en vue de déterminer la taille d'implant optimale parmi plusieurs tailles disponibles.

Suivant l'invention, le procédé est caractérisé en ce qu'il comporte l'étape qui consiste, une fois la taille en hauteur dans le plan antéro-postérieur déterminée, à faire coulisser un coulisseau afin qu'un premier pointeur indique la taille sélectionnée et, dans le même temps, qu'un deuxième pointeur indique l'épaisseur en hauteur du plan de coupe au niveau des condyles postérieurs pour un ajustement parfait au niveau de la corticale antérieure de l'implant de taille choisie.

On décrit aux figures des dessins un mode de réalisation d'un mesureur suivant l'invention, à titre non limitatif uniquement.

La figure 1 est une vue de face d'un mesureur suivant l'invention.

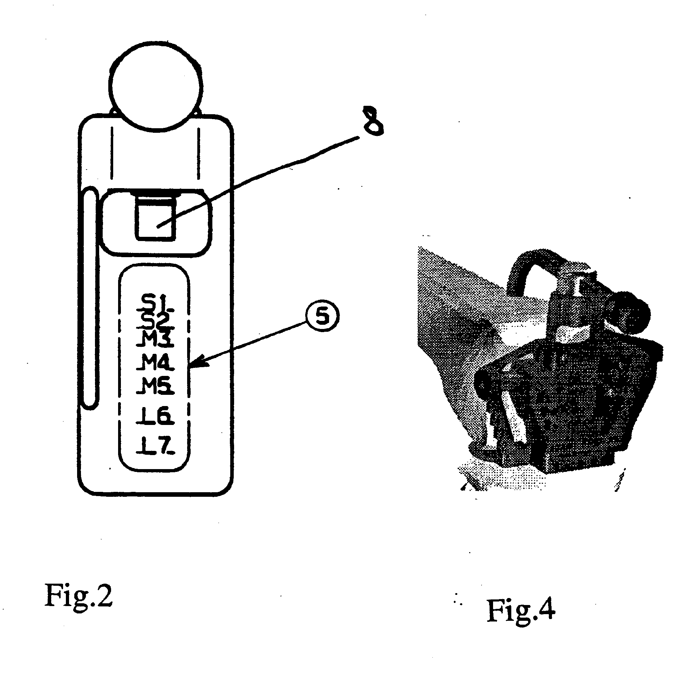

La figure 2 est une vue d'une partie du mesureur de la figure 1, à savoir le palpeur et son support gradué associé.

La figure 3 est une vue en perspective du mesureur de la figure 1.

La figure 4 est une vue en perspective montrant le mesureur de taille installé sur l'extrémité distale d'un fémur, dont la coupe distale a déjà été faite.

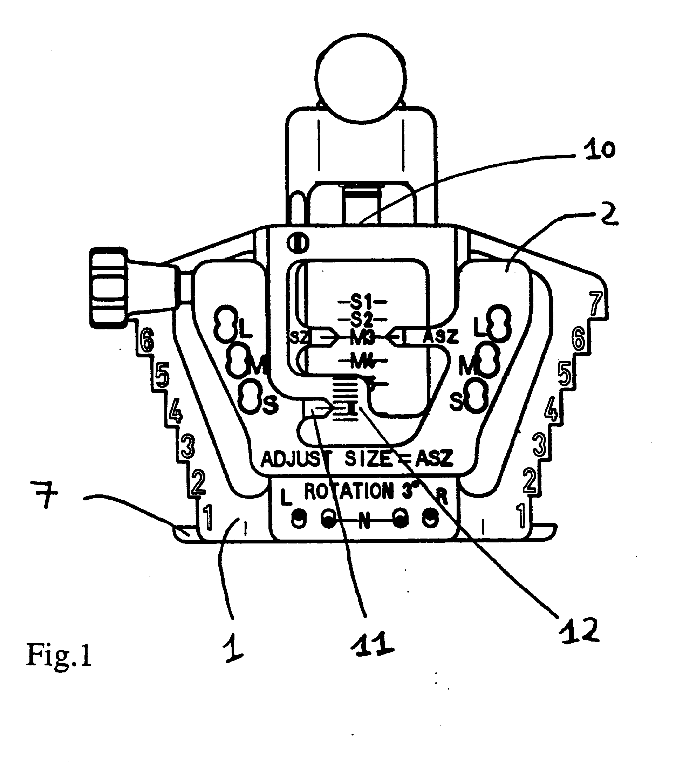

Comme on le voit aux figures, le mesureur est constitué d'un corps principal 1. Ce corps 1 principal comporte un plateau 7 de base destiné à être mis en contact avec les condyles du fémur (voir la figure 4). Un ensemble 4 palpeur est monté mobile en translation verticale à la figure 1 (la direction verticale étant ici la direction définie par l'intersection des plans médio-latéral et antéro-postérieur) et comporte, outre un palpeur 8, une plaque 5 portant des repères S1, S2, M3, M4, M5, L6, L7, correspondant aux différentes tailles disponibles d'implant fémoral.

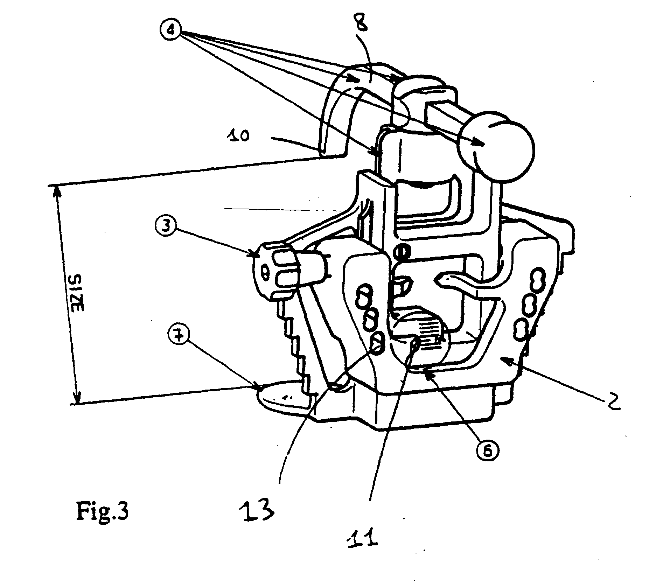

Le corps principal 1 comporte en outre un pointeur SZ principal dont la distance verticale (à la figure 3) au plateau 7 est réglée au préalable, en tenant compte également de la hauteur totale de la plaque 5, pour indiquer la taille d'implant correspondant à une position de la pointe 10 du palpeur 8.

Ainsi, une fois la pointe du palpeur en contact ou juste en affleurement avec la corticale du fémur, le pointeur SZ indique la taille exacte de l'implant qui serait nécessaire. Il est à noter qu'en général le pointeur ne se trouvera pas en face précisément d'un repère S, M ou L mais entre deux repères de ce genre.

Sur le corps principal, il est monté en outre un coulisseau 2, dont le coulissement par rapport au corps principal est libre mais peut être bloqué par une clé 3 à molette. Ce coulisseau comporte un premier pointeur ASZ et un deuxième pointeur 11. Les dimensions du coulisseau, et notamment les distances mesurées verticalement à la figure 3 entre le pointeur ASZ et les trous 13, sont choisies en fonction des dimensions des tailles d'implants possibles (S1, ..., L7) disponibles sur le mesureur, pour faire en sorte que le deuxième pointeur, lorsque le premier pointeur se déplace dans la plage utile (c'est-à-dire en face de la région de la plaque 5 comportant les repères S1, ..., L7), le deuxième pointeur se déplace en face d'une région 6 fixe par rapport au corps principal, notamment sur le corps principal, cette région comportant des gradations 12, ici en millimètres, pour indiquer le décalage par rapport à la taille choisie, décalage correspondant à la hauteur de résection à réaliser au niveau postérieur lorsque l'on définit la taille d'implant sélectionnée pour affleurer parfaitement avec la corticale inférieure.

Le coulisseau comporte en outre des séries de trous 13, ici regroupés par tailles S, M et L qui permette le positionnement parfait de pointes qu'on y insère pour percer dans l'os les points précis de fixation d'un guide de coupe pour réaliser les coupes antérieure, postérieure, chanfrein antérieur et chanfrein postérieur, correspondant au choix de la taille d'implant sélectionnée par l'utilisation précédente du mesureur.

## Revendications

1. Mesureur de taille, comportant un corps (1) principal ayant un palpeur (8) agencé de manière mobile par rapport au corps principal, de sorte qu'un pointeur SZ principal soit déplacé en fonction du réglage en face d'une plage comportant plusieurs repères (S1, S2, M3, M4, M5, L6, L7) correspondant aux tailles d'implant disponibles, et un coulisseau monté mobile par rapport au corps principal, le coulisseau comportant un premier pointeur (ASZ) et un deuxième pointeur (11), le premier pointeur étant agencé de manière à être déplacé, lors du coulissement du coulisseau (2), en face de ladite plage, **caractérisé en ce que** le coulisseau comporte un deuxième porteur, le deuxième pointeur se déplaçant, lors du mouvement du coulisseau, en face de gradations (12) disposées sur une région (6) fixe par rapport au corps principal, la distance entre les deux pointeurs du coulisseau étant telle que lorsque le premier pointeur est en face d'un des repères correspondant à l'une des tailles d'implant disponibles, le deuxième pointeur indique sur les gradations (12) la hauteur, par exemple en millimètres, de la coupe à réaliser en postérieur afin que l'implant affleure parfaitement au niveau de la corticale antérieure.

2. Mesureur suivant la revendication 1, **caractérisé en ce que** la plage se trouve sur une plaque (5), solidaire du palpeur (8) et sensiblement de forme plane pour s'adapter à un fémur dont la coupe distale a déjà été réalisée.

3. Mesureur suivant la revendication 1 ou 2, **caractérisé en ce que** le corps principal comporte un plateau (7) de réception des condyles, disposé en face du palpeur (8).

## Claims

1. A size measurer, comprising a main body **(1)** with a sensor-head **(8)** not fixed to the main body, so that a main pointer **SZ** is moved in accordance with the adjustment opposite a range which includes several markers (S1, S2, M3, M4, M5, L6, L7) corresponding to the sizes of the available implants, and a slider fitted separately in relation to the main body, the slider including a first pointer **(ASZ)** and a second pointer **(11),** the first pointer being fitted in a such a way as to be moved, after the sliding of the slider **(2),** opposite the said range, **characterised by** the fact that the slider incorporates a second pointer, the second pointer being moved, after the movement of the slider, opposite the gradations **(12)** positioned on a fixed area **(6)** in relation to the main body, the distance between the two points of the slider being such that when the first pointer is opposite the markers corresponding to one of the sizes of the available implants, the second pointer indicates by the gradations **(12)** the height, in millimetres for example, of the posterior cut to be made in order that the implant is perfectly flush with the level of the anterior cortical.

2. A measurer according to claim 1, **characterised by** the fact that the range is positioned on the plate **(5),** interdependant of the sensor-head **(8)** and roughly level to be adapted to a femur when the distal cut has already been made.

3. A measurer according to claim 1 or 2, **characterised by** the fact that the main body includes a plateau **(7)** to receive the condyles, positioned opposite to the sensor-head **(8).**

## Patentansprüche

1. Größenmessgerät, umfassend einen Hauptkörper (1) mit einem Fühler (8), der beweglich in Bezug auf den Hauptkörper angeordnet ist, so dass ein Hauptzeiger SZ als Funktion der Einstellung auf einem Skalenbereich bewegt wird, der mehrere den verfügbaren Implantatgrößen entsprechende Skalenpunkte (S1, S2, M3, M4, M5, L6, L7) aufweist, und einen Schieber, der bezüglich des Hauptkörpers beweglich montiert ist, wobei der Schieber einen ersten Zeiger (ASZ) und einen zweiten Zeiger (11) umfasst, wobei der erste Zeiger so angeordnet ist, dass er während des Verschiebens des Schiebers (2) auf dem genannten Skalenbereich bewegt wird, **dadurch gekennzeichnet, dass** der Schieber einen zweiten Zeiger umfasst, wobei der zweite Zeiger sich, während der Bewegung des Schiebers, auf Gradierungen (12), die angebracht sind auf einer bezüglich des Hauptkörpers feststehenden Region (6), bewegt, wobei der Abstand zwischen den zwei Zeigern des Schiebers derartig ist, dass wenn der erste Zeiger auf einem der Skalenpunkte, entsprechend einer der verfügbaren Implantatgrößen, steht, der zweite Zeiger auf den Gradierungen (12) die Höhe, zum Beispiel in Millimetern, des Schnitts anzeigt, der posterior vorgenommen werden muss, damit das Implantat der Ebene der anterioren Kortikalis perfekt angeglichen wird.

2. Messgerät gemäß Anspruch 1, **dadurch gekennzeichnet, dass** sich der Skalenbereich auf einer Plakette (5) befindet, welche am Fühler (8) befestigt und im wesentlichen von flacher Form ist, um sich an einen Femur anzupassen, dessen Distalschnitt bereits durchgeführt worden ist.

3. Messgerät gemäß Anspruch 1 oder 2, **dadurch gekennzeichnet, dass** der Hauptkörper einen Teller (7) zum Aufnehmen der Kondylen umfasst, welcher gegenüberliegend zum Fühler (8) angeordnet ist.
